Europäisches Patentamt

European Patent Office

Office européen des brevets

(11) Veröffentlichungsnummer: **0 296 349**

**A2**

(12) **EUROPÄISCHE PATENTANMELDUNG**

(21) Anmeldenummer: 88107572.5

(22) Anmeldetag: 11.05.88

(30) Priorität: 24.06.87 DE 3720826

(43) Veröffentlichungstag der Anmeldung:
28.12.88 Patentblatt 88/52

(84) Benannte Vertragsstaaten:
CH DE FR GB LI

(51) Int. Cl.⁴ **A61B 17/22**

(71) Anmelder: **DORNIER MEDIZINTECHNIK GMBH**
**Postfach 1128**
**D-8034 Germering 1(DE)**

(72) Erfinder: **Erhardt, Wolfgang, Dipl.-Ing.**
**Landsberger Strasse 61**
**D-8080 Fürstenfeldbruck(DE)**
Erfinder: **Grözinger, Reiner, Dipl.-Ing.**
**Greppenstrasse 9**
**D-8031 Alling(DE)**
Erfinder: **Wess, Othmar, Dr. Dipl.-Phys.**
**Max- Nadler Strasse 17**
**D-8000 München 81(DE)**

(74) Vertreter: **Landsmann, Ralf, Dipl.-Ing.**
**c/o DORNIER GMBH Postfach 1420**
**D-7990 Friedrichshafen 1(DE)**

(54) **Atemtriggerung.**

㊾ Vorrichtung zur atemgetriggerten Einleitung von Stosswellen in den Körper (PK) von Lebewesen, mit einem Druckaufnehmer (pr) im Ankoppelkissen (K) und einer Auswerteschaltung (AT), die den von der Atembewegung verursachten Druckverlauf zur Stosswellenfreigabe benutzt.

Fig.1

## Atemtriggerung

Die Erfindung betrifft eine Vorrichtung zur atemgetriggerten Einleitung von Stosswellen in den Körper von Lebewesen nach dem Oberbegriff des Anspruchs 1.

Aus der DE-PS 31 46 628 ist eine Vorrichtung zur Zerkleinerung von Konkrementen bekannt, bei der Stosswellen atemgetriggert ausgelöst werden. Als Meßwertaufnehmer ist ein Spirometer vorgeschlagen. Die Möglichkeit der gleichzeitigen Triggerung mit dem EKG ist ebenfalls vorgeschlagen.

Aus der DE-PS 31 46 626 ist eine Vorrichtung zur Zerkleinerung von Konkrementen bekannt, die ein Ankoppelkissen für die Stosswellen aufweist.

Aufgabe der Erfindung ist es, eine Vorrichtung zur atemge triggerten Einleitung von Stosswellen zu schaffen, die das Spirometer ersetzt.

Diese Aufgabe wird erfindungsgemäß gelöst von einer Vorrichtung mit den Merkmalen des Anspruchs 1. Vorteilhafte Ausgestaltungen der Erfindung sowie Verwendungen sind Gegenstände von Unteransprüchen.

Die Erfindung bietet folgende Vorteile:
Die Erfassung der Atembewegung für die Stosswellenauslösefunktionen und -vorrichtungen bei Stosswellenapplikationen erfolgt mit bekannten Systemkomponenten (Kissen, Drucksensor), wodurch keine zusätzlichen Meßwertaufnahmevorrichtungen für die Atmung (z.B. Spirometer) erforderlich sind. Mit dem Ankoppeln des Kissens liegt bereits das Atemtriggersignal vor. Besondere Verbindungen mit dem Patienten, Meßgeräte, Leitungen etc., sind nicht notwendig.

Erfindungsgemäß wird also am bereits vorhandenen Einkoppelkissen lediglich ein Drucksensor angebracht und dessen Signale in einer einfachen Elektronik ausgewertet. Dies ersetzt eigene Verbindungen oder eigene Meßwertaufnehmer.

In einer Ausführungsform wird der Drucksensor zur Regelung der Niveaueinstellung und/oder zur Konstanthaltung des An koppeldrucks verwendet. Vorteilhaft ist ein Regler, bei dem der Nullbereich extern einstellbar ist. Der Nullbereich kann dann der Amplitude der atembedingten Druckänderung angepasst werden. Als Regler können an sich bekannte Zwei- oder Dreipunktregler, Zweischrittregler, Schalter oder Dreipunktschrittregler mit PID-Verhalten oder ähnliche Regler verwendet werden.

In einer Ausführungsform kann das Atemtriggersignal auch zur Freigabe der Aufnahmen des Röntgendiagnostiksystems herangezogen werden.

In einer anderen Ausführung wird neben der Atemtriggerung auch eine Triggerung mit dem EKG des Patienten durchgeführt.

Ein Ausführungsbeispiel der Erfindung wird anhand dreier Figuren erläutert.

Figur 1 zeigt eine erfindungsgemäße Vorrichtung,

Figur 2 zeigt den atembedingten Druckverlauf und Schaltzustände einer erfindungsgemäßen Regelung,

Figur 3 zeigt ein Blockschaltbild einer Ausführung mit EKG- und Atemtriggerung.

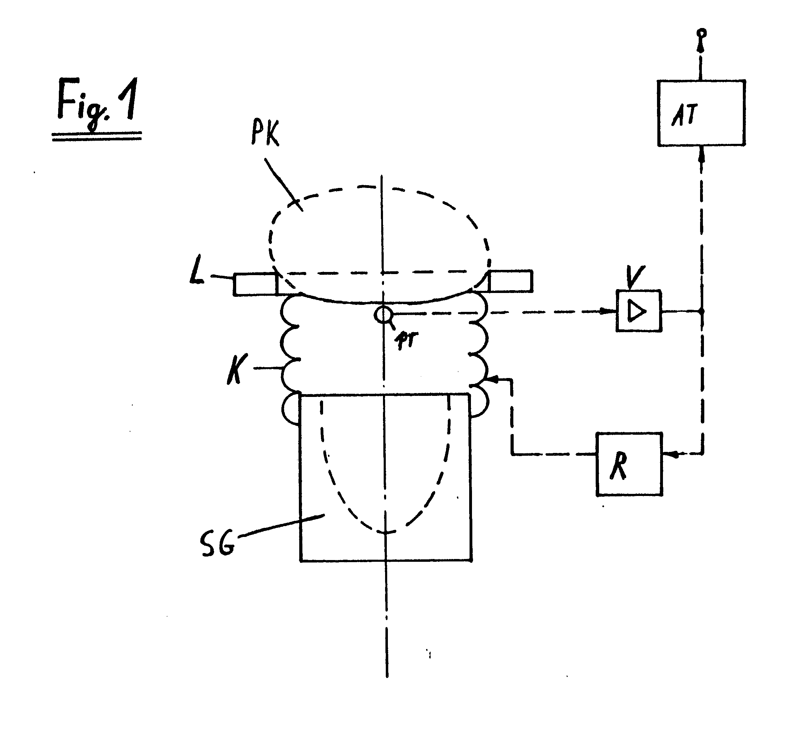

Figur 1 zeigt eine Ausführung der Erfindung, bei der neben der Atemtriggerung auch die Niveaueinstellung des Ankoppelkissens K durchgeführt wird.

Auf einer Liege L liegt der Patientenkörper PK. Darunter ist der Stosswellengenerator SG angeordnet, der fokussierte Stosswellen emittiert. Die Stosswellen werden über das Ankoppelkissen K in den Patientenkörper PK geleitet. Am Ankoppelkissen K ist ein Druckaufnehmer (Drucksensor) pr vorgesehen, dessen Signale über den Verstärker V zur Auswerteschaltung (Atemtrigger AT) geleitet werden. Die Auswerteschaltung AT ist mit dem Druckregler R verbunden, der den Druck im Ankoppelkissen K regelt.

Die Ankoppelung des mit dem Koppelmedium gefüllten Ankoppelkissen K an den Patientenkörper PK erfolgt durch das Befüllen und Entleeren des Ankoppelkissens K mit einem Koppelmedium, z.B. Wasser. Für die Niveaueinstellung des Ankoppelkissens K und die Konstanthaltung des Ankoppeldrucks ist eine Druckregelung für den Druck des Koppelmediums innerhalb des Ankoppelkissens K vorgesehen. Der Druck des Koppelmediums wird für die Druckregelung mit einem Drucksensor pr detektiert und dessen Ausgangssignal über die Auswerteschaltung AT an den Druckregler R geführt.

Der elektronische Drucksensor wird erfindungsgemäß gleichzeitig zur Erfassung von Druckänderungen im Koppelmedium des Kissens K aufgrund der Atembewegungen des Patientenkörpers PK genutzt.

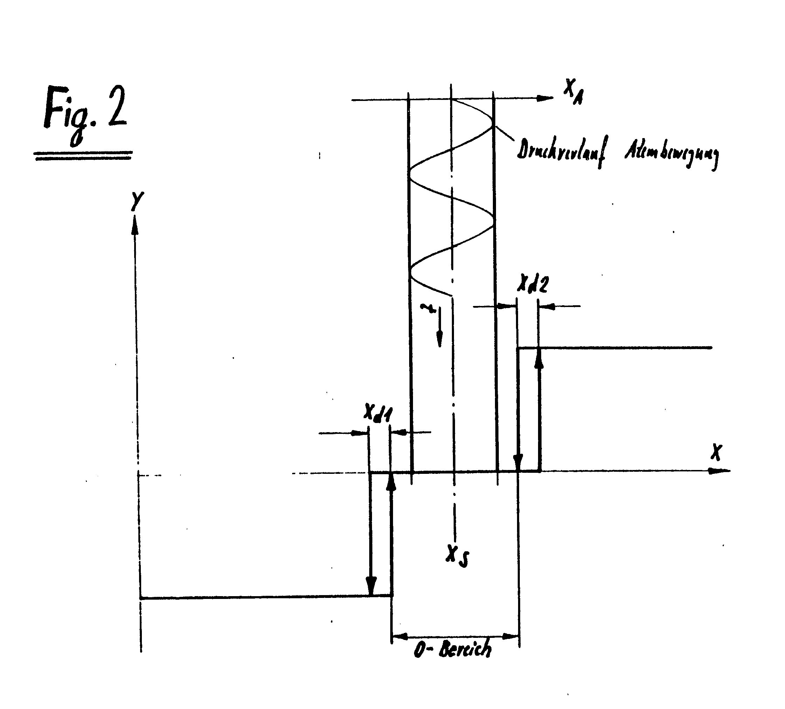

Figur 2 zeigt die Abhängigkeit der Stellgröße y (Füllen oder Entleeren) von der Regelgröße x (Druck im Kissen). Bei Anliegen des Ankoppelkissens K am Patientenkörper PK steigt z.B. der Druck und der Regler R stoppt die Flüssigkeitszufuhr.

$x_A$ ist der durch die Atembewegung verursachte Druck verlauf.

$x_S$ bezeichnet den Sollwert (Kissen K am Patientenkörper PK anliegend).

$x_{d1}$ ist die erste Schaltdifferenz des Reglers R.

$x_{d2}$ ist die zweite Schaltdifferenz des Reglers R.

t bezeichnet die Zeit.

Der Druckregler R arbeitet jedoch mit einer bestimmten Schalthysterese (Nullbereich), innerhalb der bei Druckänderungen im Koppelmedium keine Nachregelung des Druckes erfolgt.

Durch die Atembewegung des Patientenkörpers PK wird der Druck im Koppelmedium des Ankoppelkissens K innerhalb bestimmter, in Figur 2 gezeigter Grenzen periodisch analog zur Atembewegung verändert. Es wurde gefunden, dass die periodischen Druckänderungen im Koppelmedium des Ankoppelkissens K ohne Phasenverschiebung und mit der gleichen Frequenz der Atembewegung verlaufen.

Erfindungsgemäß wird der Nullbereich so eingestellt, dass die Amplitude der periodischen Druckänderungen durch die Atembewegungen innerhalb der Schalthysterese (Nullbereich) der Druckregelung liegt, so dass durch diese Druckänderung die Druckregelung nicht aktiviert wird.

Die Frequenz, Phase und Amplitude der periodischen Druckänderungen durch die Atembewegungen werden somit während der normalen Stosswellenapplikationsphase (Ankoppelkissen K ist im angekoppelten Zustand) nicht gestört und entsprechen dem tatsächlichen Verlauf der Atembewegungen. Diese periodischen Druckänderungen durch die Atembewegung werden erfingungsgemäß vom Drucksensor pr erfasst und einer elektronischen Einrichtung (Auswerteschaltung AT) zugeführt. Mit deren Hilfe wird dann die Stosswelle ausgelöst, wobei als weiteres Triggersignal das EKG verwendet werden kann.

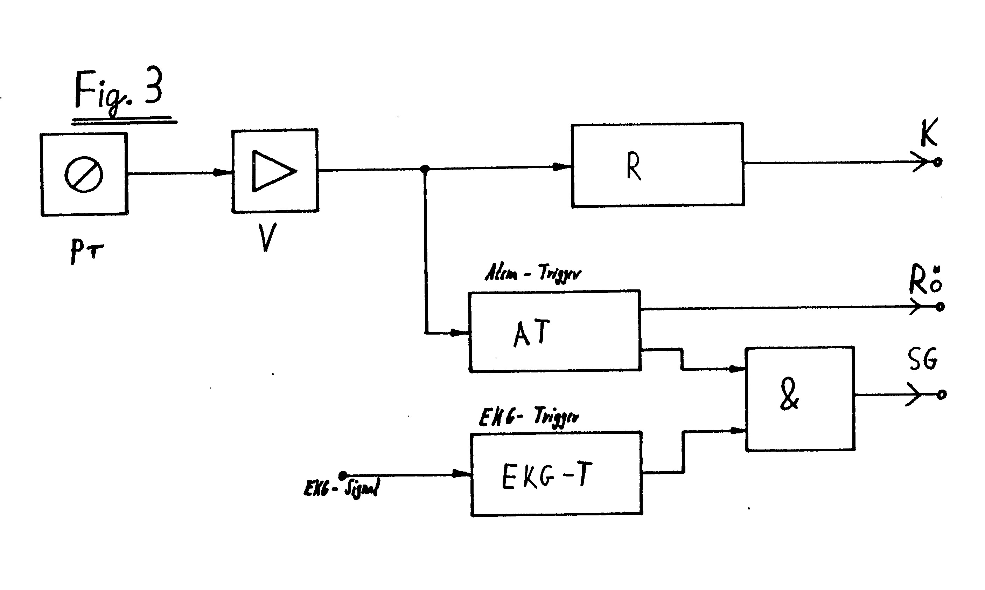

Figur 3 zeigt eine solche Auswerteschaltung mit Drucksensor pr, Verstärker V und Druckregler R, dessen Ausgang zum Ankoppelkissen K führt. Vorgesehen sind weiter die Auswerteschaltung AT für die Atemtriggerung und eine Auswerteschal tung EKG-T für die EKG-Triggerung, die über eine logische UND-Schaltung (UND-Gatter, UND-Zeichen) zur Stosswellenquelle SG führen und dort die Stosswellen gerade passend zur Atemphase und zu einer EKG-Phase freigeben. Eine zweite Verwendung des Atemtriggersignals zur Freigabe der Röntgendiagnostikanlage (Rö) ist ebenfalls gezeigt. Der Vorteil an dieser Anordnung ist, dass alle Röntgenbilder stets bei gleicher Atemphase, das heißt bei gleicher Stellung des Brustkorbs und somit bei gleicher Lage der Behandlungsstelle (z.B. des zu zertrümmernden Konkrements), aufgenommen werden.

mer (pr) im Ankoppelkissen (K) und eine Auswerteschaltung (AT), die den von der Atembewegung verursachten Druckverlauf zur Stosswellenfreigabe benutzt.

2. Vorrichtung nach Anspruch 1, gekennzeichnet durch einen elektronischen Drucksensor als Druckaufnehmer (pr).

3. Vorrichtung nach einem der vorhergehenden Ansprüche, gekennzeichnet durch eine Auswerteschaltung (AT), die zugleich die Niveaueinstellung und/oder die Konstanthaltung des Ankoppeldrucks regelt.

4. Vorrichtung nach Anspruch 3, dadurch gekennzeichnet, dass der Druckregler (R) einen einstellbaren Nullbereich hat, der breiter einstellbar ist als ca. 10 mbar.

5. Vorrichtung nach Anspruch 3, dadurch gekennzeichnet, dass der Druckregler (R) ein Zweipunktregler, ein Zweischrittregler, ein Dreipunktregler oder ein Dreipunktschrittregler mit PID-Verhalten ist.

6. Vorrichtung nach einem der vorhergehenden Ansprüche, gekennzeichnet durch eine Verwendung des Signals zur Röntgenfreigabe.

7. Vorrichtung nach einem der vorhergehenden Ansprüche, dadurch gekennzeichnet, dass die Stosswellenauslösung zusätzlich vom EKG getriggert wird.

8. Verwendung eines Druckaufnehmers (pr) im Ankoppelkissen (K) bei der Stosswellentherapie zur Atemtriggerung.

9. Triggerung der Stosswellenauslösung für therapeutische Zwecke durch Aufnahme der atembedingten Druckänderung im Ankoppelkissen (K).

10. Triggerung der Stosswellen nach Anspruch 9, dadurch gekennzeichnet, dass das EKG als zweites Triggersignal verwendet wird.

11. Triggerung der Röntgendiagnostikanlage durch die atembedingte Druckänderung im Ankoppelkissen (K).

**Ansprüche**

1. Vorrichtung zur atemgetriggerten Einleitung von Stosswellen in den Körper (PK) von Lebewesen, **gekennzeichnet** durch einen Druckaufneh-

Fig. 1

PK

L

V

AT

K

pr

R

SG

EP 0 296 349 A2

Fig. 2

Fig. 3

PT  V  R → K

Alarm - Trigger

AT → Rö

EKG - Trigger

EKG-Signal → EKG-T

&  → SG

EP 0 296 349 A2